# EUROPÄISCHE PATENTSCHRIFT

(11) **EP 2 543 309 B1**
(45) Veröffentlichungstag und Bekanntmachung des Hinweises auf die Patenterteilung: **15.02.2017**
(21) Anmeldenummer: 12175010.3
(22) Anmeldetag: 04.07.2012
(51) Int. Cl.: A61B 1/06, A61B 1/00, A61B 1/05, H04B 10/00, G02B 23/26, A61B 1/04

(54) **Endoskopische Anordnung**
Endoscopic assembly
Agencement endoscopique

(30) Priorität: 04.07.2011 DE 102011106386
(43) Veröffentlichungstag der Anmeldung: 09.01.2013
(73) Patentinhaber: Karl Storz GmbH & Co. KG, 78532 Tuttlingen (DE)
(72) Erfinder: Baum, Eckhart, 78589 Dürbheim (DE); Irion, Klaus-Martin, 78576 Emmingen-Liptingen (DE); Schwarz, Peter, 78532 Tuttlingen-Nendingen (DE); Efinger, Daniel, 78554 Aldingen (DE)
(74) Vertreter: Witte, Weller & Partner Patentanwälte mbB

(56) Entgegenhaltungen:
- DE-C2- 19 947 811
- JP-A- 2006 051 162
- JP-A- 2006 181 021
- US-A- 4 679 448
- US-A1- 2008 039 686

## Beschreibung

Die vorliegende Erfindung betrifft eine endoskopische Anordnung mit einem ersten distalen Ende, an dem eine bildgebende Einrichtung angeordnet ist, und einem ersten proximalen Ende zum Anschluss an eine elektrische Einheit, mit einem Datenübertragungselement, das als Lichtleiter ausgebildet ist und ein zweites distales Ende und ein zweites proximales Ende aufweist, und mit einem elektrooptischen Wandler, der dafür ausgebildet ist, digitale Daten, die von der bildgebenden Einrichtung erzeugt werden, in ein optisches Signal zu wandeln, und das Datenübertragungselement an seinem zweiten distalen Ende optisch mit dem elektrooptischen Wandler gekoppelt ist, um das optische Signal in das Datenübertragungselement einzukoppeln.

Eine solche endoskopische Anordnung ist aus DE 199 47 811 C2 bekannt.

Bei endoskopischen Anordnungen, insbesondere bei Endoskopen und videoassistierten endoskopischen Einheiten, besteht ein ständiger Bedarf an besser auflösenden, insbesondere hochauflösenden, und farbtreuen Systemen. Betroffen sind davon bekanntermaßen endoskopische Kameras, die proximalseitig oft an starre Endoskope gekoppelt werden. Wie später noch erläutert wird, widmet sich die Erfindung aber auch den meist flexiblen Videoendoskopen, die bildgebende Einrichtungen an ihrer Spitze tragen. Zudem werden auch endoskopische Kapseln berücksichtigt, die autonom durch den Magen-Darm-Trakt passieren und dabei ihre Bildinformationen übertragen. Die Übertragung bei endoskopischen Kapseln findet gemäß dem Stand der Technik drahtlos statt. Teilweise sind endoskopische Kapseln auch mit einem Aufzeichnungsmedium für eine spätere Betrachtung der Bildinformation ausgestattet worden. Neben medizinischen Applikationen werden videoassistierte endoskopische Einheiten auch in verschiedenen technischen Applikationsfeldern, wie der Rohrinspektion, Triebwerkinspektion oder Motoreninspektion, etc. eingesetzt.

Bei den Ausführungsformen gemäß dem Stand der Technik existieren trotz aller Anstrengungen Probleme mit hoch- bzw. höchstauflösenden Bildsensoren in Verbindung mit der Bildübertragung bei Verwendung von dünnkalibrigen Durchmessern mit endoskopischen Übertragungslängen, die je nach endoskopische Applikation variieren. Insbesondere bei endoskopischen Kameras und Videoendoskopen besteht die Anforderung nach möglichst dünnkalibrigen und leichten Übertragungssystemen, einschließlich der Kabel, die einen breiten Bereich an möglichen Kabellängen erlauben. Bisher existiert kein kleinkalibriges Videoendoskop mit einer so genannten Full-HD-Auflösung im Bereich von zwei Millionen Pixeln. Endoskopische Kameras mit Full-HD-Auflösung existieren zwar, doch sind die Kabeldurchmesser relativ groß und schwer und damit für den endochirurgischen Einsatz etwas unhandlich.

Besonders problematisch ist die Situation bei endoskopischen Kapseln, die nach heutigem Stand nur eine schlechte Bildauflösung und nur eine geringe Bildwiederholrate bieten können. Dies liegt im Wesentlichen darin begründet, dass die drahtlose Übertragung von höheren Bandbreiten durch menschliches Gewebe nicht möglich ist. Stattet man die endoskopische Kapsel mit einem Speicher aus, so sind selbst bei den heute verfügbaren Speichermedien die Kapazität des Speichers und der zur Verfügung stehende Bauraum schnell erschöpft, wenn man ein Full-HD-Video mit einer Bildwiederholfrequenz von 25 Hz oder 30 Hz oder mehr speichern möchte.

Endoskopische Anordnungen mit distalseitigen oder proximalseitigen elektronischen Bildaufnehmersystemen verwenden gemäß dem Stand der Technik aufwändige und teure Kabelsysteme, um die Bildinformation vom Endoskop in eine fern vom Patienten stehende Kontrolleinheit zu übertragen, sowie Kontrollinformation und elektrische Energie von der Kontrolleinheit zum Bildaufnehmersystem zu übertragen. Ziel bei der Erhöhung der Bildauflösung ist es, dem Mediziner ein möglichst natürliches endoskopisches Bild mit hoher Auflösung für seine Intervention zu bieten. Die unmittelbare Konsequenz aus der höheren Pixelanzahl der Bildsensoren sind immer höhere Signal-Übertragungsfrequenzen. Um die Signaldämpfung bei höheren Übertragungsfrequenzen zu reduzieren und die effektive Bandbreite zu erhöhen, sind größere Kabeldurchmesser erforderlich. Neben der Zunahme des Gewichts stellt aber auch die elektromagnetische Verträglichkeit (EMV) ein größeres Problem dar.

Bei Videoendoskopen ergibt sich zudem das Problem, dass eine Erhöhung des Kabeldurchmessers kaum möglich ist, da dies zu Lasten einer Erhöhung des Gesamtdurchmessers oder zu einer Verringerung des Durchmessers von anderen notwendigen endoskopischen Komponenten wie Saugkanälen, Lichtleitern etc. gehen würde.
Eine weitere Anforderung, die an endoskopische Kameras und an Videoendoskope gestellt wird, ist, dass die Kabelsysteme am Endoskop bzw. an der proximalseitigen Kamera zu Reinigungs- und Sterilisationszwecken eine einfach lösbare Verbindung aufweisen sollten. Da die Bildaufnehmersysteme häufig einge-setzt werden, sind die Kabelsysteme als Verschleißartikel anzusehen und müssen einfach repariert bzw. ersetzt werden können. Da die Systeme über einen mehradri-gen komplexen Aufbau verfügen, stellt das Kabelsystem mit seinen Anschlüssen einen nennenswerten Kostenanteil am Gesamtsystem dar. Es ist daher auch ein Ziel, die Gestehungskosten des Kabelsystems zu reduzieren.
JP 2006-181021 zeigt eine endoskopische Einrichtung, bei der Bilddaten von einem distalen Ende über ein optisches Kabel an ein proximales Ende übertragen werden. Das Bildsignal wird distalseitig von einem Parallel/Seriell-Wandler serialisiert, seriell über das optische Kabel übertragen und schließlich proximalseitig mittels eines Seriell/Parallel-Wandlers parallelisiert. Bei einer Ausführungsform wird aus einem linken und einem rechten optischen System ein 3D-Signal erzeugt, das über das optische Kabel übertragen wird.
US 4,679,448 zeigt ein System zum inneren Inspizieren von Pipelines. Das System weist einen fahrbaren Roboter und eine Kontrollstation auf. Der Roboter und die Kontrollstation sind über ein Glasfaserkabel miteinander verbunden. Ein Bildsignal, das der Roboter erzeugt, wird mittels eines elektro-optischen Wandlers in ein optisches Signal umgesetzt, über das optische Kabel übertragen und in der Kontrollstation schließlich wieder in ein elektrisches Signal umgewandelt.
US 2008/0039686 A1 zeigt ein Endoskop-System, bei dem elektrische Signale für eine Übertragung in optische Signale umgewandelt und schließlich wieder in ein elektrisches Signal zurückgewandelt werden. Es wird gezeigt, dass verschiedene Wellenlängen innerhalb des optischen Kabels verwendet werden können. Die Signale verschiedener Wellenlängen werden mittels eines Spektralfilters getrennt.

Es ist eine Aufgabe der vorliegenden Erfindung, eine verbesserte endoskopische Anordnung aufzuzeigen, die bei geringem Durchmesser des Übertragungssystems, geringen Dämpfungsverlusten und hohen Übertragungsfrequenzen auch große Leitungslängen von 5 m und mehr bei geringem Gewicht und günstigen Kosten ermöglicht. Die endoskopische Anordnung soll eine geringe EMV-Abstrahlung haben und sowohl bei endoskopischen Kameras, Videoendoskopen und endoskopischen Kapseln einsetzbar sein.

Die Aufgabe wird gelöst durch eine endoskopische Anordnung der eingangs genannten Art, wobei die bildgebende Einrichtung mindestens zwei Datenausgänge mit jeweils einem digitalen Ausgangssignal aufweist und zwischen der bildgebenden Einrichtung und dem elektrooptischen Wandler ein erster Signalwandler angeordnet ist, der dafür ausgebildet ist, die digitalen Ausgangssignale zu einem digitalen kombinierten Ausgangssignal zusammenzuführen.

Eine Besonderheit der Erfindung liegt darin, dass die digitalen Daten von mindestens zwei Datenausgängen nahezu gleichzeitig übertragen werden können, ohne dafür mehrere parallele Leitungen zu benötigen. Um dies zu erzielen, gelangen die digitalen Ausgangssignale in einer parallelen Anordnung zum Signalwandler und werden dort zu einem digitalen kombinierten Ausgangssignal zusammengeführt. Bei einer bevorzugten Ausführungsform werden die digitalen Ausgangssignale mittels eines Multiplexers serialisiert.

Die Ausgangssignale der bildgebenden Einrichtung sollen als serielle Signale verstanden werden. Sollte ein Datenausgang bereits über parallele Ausgangssignale verfügen, die Bilddaten übertragen, so werden die einzelnen Signale der parallelen Signale jeweils wieder als serielles Signal verstanden. Unter dem Begriff "parallel" soll dabei insbesondere verstanden werden, dass mehrere Ausgangssignale gleichzeitig Nutzdaten mit Bildinformation tragen können.

Bei der bildgebenden Einrichtung handelt es sich um einen Bildaufnehmer mit mindestens zwei Datenausgängen, insbesondere Ausgängen für Daten und Timinginformation, oder um mindestens zwei Bildaufnehmer mit jeweils mindestens einem Datenausgang, insbesondere d.h. Ausgang mit kombiniertem Daten-/Taktsignal. Im ersten Fall kann es sich um einen Bildsensor handeln, der eine Bildfläche aufweist, die zum Auslesen der Bilddaten aber in mindestens zwei Teilflächen unterteilt ist, deren Informationen jeweils über einen eigenen Datenausgang mit kombiniertem Daten- und Taktsignal gesendet werden. Es kann sich allerdings auch um einen Bildsensor handeln, der einen ersten Datenausgang mit einem reinen Datensignal und einen zweiten Datenausgang mit einem Taktsignal aufweist. Im zweiten Fall kann es sich insbesondere um mindestens zwei Bildsensoren mit getrennten Bildflächen handeln, wobei jeder Bildsensor über jeweils mindestens einen Datenausgang mit kombiniertem Daten-/Taktsignal verfügt.

Der elektrooptische Wandler und der Signalwandler können jeweils diskret ausgeführt sein, sind aber bevorzugt in einem Bauteil kombiniert. Der elektrooptische Wandler ist bevorzugt als Laserdiode ausgeführt, insbesondere als VCSEL-Diode (Vertical-Cavity Surface-Emitting Laser). Inbesondere kommen dabei genau eine Laserdiode oder maximal zwei Laserdioden zum Einsatz, da dies den benötigten Bauraum gering hält.

Die bildgebende Einrichtung weist bevorzugt ein Objektiv auf, bzw. der bildgebenden Einrichtung ist bevorzugt ein Objektiv zugeordnet. Die Ausgestaltung des elektrooptischen Wandlers und des Signalwandlers ist bevorzugt so ausgeführt, dass Signale bidirektional von der bildgebenden Einrichtung weg und zur bildgebenden Einrichtung hin gesendet und empfangen werden können. Dies ermöglicht es auf einfache Weise, das Datenübertragungselement gleichzeitig für eine Übertragung von Steuersignalen in Richtung der bildgebenden Einrichtung zu nutzen. Es ist aber ebenso möglich, am ersten distalen Ende einen optoelektrischen Wandler für die Auskopplung von Steuersignalen am ersten distalen Ende einzusetzen. Die Wellenlänge des von dem elektrooptischen Wandler erzeugten Lichts liegt bevorzugt zwischen 700 nm und 1550 nm.

Als Ausgangssignal bzw. Datensignal soll allgemein ein Signal angesehen werden, das ausgehend vom Bildsensor Bilddaten enthält. Das Ausgangssignal muss aber nicht auf reine Bilddaten beschränkt sein, sondern kann auch Timinginformation oder andere Daten beinhalten, die die Bilddaten beschreiben. Es wird als vorteilhaft angesehen, wenn die Datenausgänge der bildgebenden Einrichtung jeweils differenziell ausgeführt sind. Die resultierenden parallelen differenziellen Signale können dann bevorzugt vor dem elektrooptischen Wandler oder im elektrooptischen Wandler zusammengefasst werden oder aber als parallele Ausgangssignale betrachtet werden, die vom Signalwandler in dem digitalen kombinierten Ausgangssignal zusammengeführt werden.

Die Erfindung ermöglicht eine Vielzahl von Vorteilen, darunter die geringe distalseitige Baugröße und den sehr geringen Durchmesser des Datenübertragungselements bei hoher Datenrate, insbesondere von mehr als 1 Gbit/s. Des Weiteren ergeben sich keine EMV-Probleme über die Leitungslänge. Im Vergleich dazu wirken elektrische Leitungen wie eine Antenne, wobei eine Abschirmung aufgrund der Platz- bzw. Durchmesserproblematik stets kritisch ist. Durch die elektrooptische Kopplung ist gleichzeitig auch eine galvanische Entkopplung gegeben, die laut Norm für medizinisch endoskopische Systeme sinnvoll ist. Zudem ist ein weiterer Vorteil, dass das System aufgrund seiner kurzen elektrischen Leitungen und der optischen Übertragung zumindest im Hinblick auf die gewünschte Informationsübertragung bei hoher Auflösung für einen Einsatz in MR-Systemen (Magnetresonanz-Systemen) geeignet ist.

Hinsichtlich des ersten Signalwandlers ist es bevorzugt, dass dieser einen Codierer und einen Multiplexer aufweist, der aus den mehreren digitalen Ausgangssignalen der bildgebenden Einheit ein kombiniertes digitales Ausgangssignal höherer Taktrate und ohne Gleichanteil erzeugt. Dafür kann insbesondere eine 8B/10B-Codierung verwendet werden, bei der jedem 8-Bit-Wort ein 10-Bit-Wort so zugeordnet wird, dass eine Gleichtaktunterdrückung bewirkt wird. Der Multiplexer arbeitet dabei vorzugsweise nach dem Zeitmultiplexverfahren, wobei sowohl eine synchrone als auch eine asynchrone Arbeitsweise möglich ist.

Bei der elektrischen Einheit handelt es sich bevorzugt um eine Versorgungseinheit bzw. um eine Kamerakontrolleinheit, die die distale Elektronik elektrisch versorgt, die bildgebende Einrichtung ansteuert und die Bildinformation weiterverarbeitet.

Bei einer vorteilhaften Ausgestaltung weist der Signalwandler einen Pufferspeicher auf, der dafür ausgebildet ist, zumindest eines der digitalen Ausgangssignale, bevorzugt alle digitalen Ausgangssignale, temporär zu speichern.

Diese Ausgestaltung bietet eine besonders hohe Flexibilität im Hinblick auf die Verarbeitung der mehreren Ausgangssignale. Insbesondere wird es dadurch ermöglicht, dass die Datenrate bei der Übertragung über das Datenübertragungselement höher ist, bevorzugt deutlich höher ist und insbesondere mindestens das Doppelte der Datenrate eines Ausgangssignals aus einem Datenausgang der bildgebenden Einrichtung beträgt. Es ist bevorzugt, dass der Pufferspeicher so groß gewählt ist, dass mindestens die Information eines Bildpixels, bevorzugt mindestens einer Reihe von Pixeln, und besonders bevorzugt mindestens ein vollständiges Bild, wie es von dem entsprechenden Datenausgang ausgegeben werden kann, gepuffert werden kann. Dadurch wird es ermöglicht, das digitale kombinierte Ausgangssignal pixelweise, zeilenweise oder bildweise zusammenzuführen. Da sich über das Datenübertragungselement sehr hohe Datenraten übertragen lassen, wird es so ohne Weiteres möglich, beispielsweise die Bildinformation von vier Bildflächen mit der vierfachen Datenrate über das Datenübertragungselement nahezu gleichzeitig zu übertragen.

Bei einer bevorzugten Ausgestaltung der Erfindung weist die bildgebende Einrichtung eine Videoauflösung von mehr als 0,75 Megapixel, bevorzugt von mehr als 1,5 Megapixel, besonders bevorzugt von mehr als 2 Megapixel und insbesondere von mehr als 3 Megapixel auf.

Diese Ausgestaltung ist vorteilhaft, da sich auf einfache Weise auch große Mengen von Bilddaten mittels der endoskopischen Anordnung übertragen lassen. Unter dem Begriff "Videoauflösung" soll insbesondere eine Auflösung verstanden werden, bei der mindestens 15 Bilder pro Sekunde, bevorzugt mindestens 25 Bilder pro Sekunde und besonders bevorzugt mindestens 30 Bilder pro Sekunde übertragen werden.

Gemäß der Erfindung ist am zweiten proximalen Ende des Datenübertragungselements ein erster optoelektrischer Wandler angeordnet, der dafür ausgebildet ist, das optische Signal aus dem Datenübertragungselement auszukoppeln und das optische Signal in digitale Daten zu wandeln, wobei mit dem ersten optoelektrischen Wandler ein zweiter Signalwandler verbunden ist, der dafür ausgebildet ist, ein digitales kombiniertes Ausgangssignal in mindestens zwei digitale Ausgangssignale aufzuteilen.

Dies ermöglicht eine besonders einfache Rückgewinnung der ursprünglichen Signale, die dem ersten Signalwandler übergeben wurden. Dass auch proximalseitig wieder von dem digitalen kombinierten Ausgangssignal gesprochen wird, soll dahingehend verstanden werden, dass das Signal, auch wenn es aufgrund der elektrooptischen und optoelektrischen Wandlungen nicht mehr dasselbe physikalische Signal ist, immer noch dieselben Informationen beinhaltet, wie das distalseitige digitale kombinierte Ausgangssignal. Als zweiter Signalwandler kommt dabei bevorzugt ein De-Multiplexer zum Einsatz.

Bei einer weiteren vorteilhaften Ausgestaltung der Erfindung ist zwischen dem elektrooptischen Wandler und dem zweiten distalen Ende des Datenübertragungselements ein Spektralteiler angeordnet.

Diese Ausgestaltung ermöglicht es auf einfache Weise, das Datenübertragungselement bidirektional zu nutzen. Wird Licht, insbesondere sichtbares Licht, von der proximalen Seite in das Datenübertragungselement eingekoppelt, so kann es mittels des Spektralteilers distalseitig von dem Licht getrennt werden, das vom elektrooptischen Wandler zum Zwecke der Informationsübertragung ausgesendet wird. Der Spektralteiler kann insbesondere dann effektiv eingesetzt werden, wenn zwischen der Wellenlänge für die Informationsübertragung und der Wellenlänge zur Übertragung von sichtbarem Licht ein hinreichend großer Abstand liegt. Dafür liegt die Obergrenze der Wellenlänge für das sichtbare Licht insbesondere bei 650 nm, bevorzugt bei 550 nm und besonders bevorzugt bei 500 nm. Die Untergrenze der Wellenlänge liegt für die Informationsübertragung bei 700 nm, bevorzugt bei 900 nm und besonders bevorzugt bei 1100 nm. Die bidirektionale Übertragung bietet einen doppelten Nutzen ohne eine Erhöhung des Durchmessers des Übertragungsmediums. Für die spektral getrennte Übertragung von Licht im sichtbaren Bereich und Informationen im infraroten, insbesondere nahinfraroten, Bereich werden bevorzugt Multimode-Fasern eingesetzt.

Bei einer weiteren bevorzugten Ausgestaltung der Erfindung ist am ersten distalen Ende der endoskopischen Anordnung ein zweiter optoelektrischer Wandler angeordnet, der dafür ausgebildet ist, Licht, das proximalseitig in das Datenübertragungselement eingekoppelt wird, in elektrische Energie zu wandeln.

Diese Ausgestaltung bietet eine gute Möglichkeit, das Datenübertragungselement bidirektional zu nutzen und unter galvanischer Entkopplung distalseitig elektrische Energie bereitzustellen, insbesondere für den Betrieb der bildgebenden Einrichtung. Dafür wird die Energie, die zumindest teilweise am ersten distalen Ende der endoskopischen Anordnung bereitgestellt werden soll, proximalseitig in das Datenübertragungselement eingespeist. Am ersten distalen Ende der endoskopischen Anordnung wird das Licht bevorzugt über einen Spektralteiler ausgekoppelt. Das für die Energieversorgung bestimmte Licht gelangt dann zum optoelektrischen Wandler, der dafür insbesondere als Solarzelle ausgeführt ist.

Bei einer weiteren vorteilhaften Ausgestaltung der Erfindung sind am ersten distalen Ende der endoskopischen Anordnung ein Austritt für sichtbares Licht und ein Lumineszenzwandler angeordnet, wobei der Lumineszenzwandler dafür ausgebildet ist, im Datenübertragungselement in Richtung der distalen Seite geführtes Licht in weißes Licht zu wandeln und an den Austritt weiterzuleiten.

Diese Ausgestaltung bietet die vorteilhafte Möglichkeit, proximalseitig eingespeistes Licht als weißes Licht zur Verfügung zu stellen und gleichzeitig eine gute spektrale Trennung zwischen dem Licht und dem für die Informationsübertragung verwendeten Licht zu ermöglichen. Wie bereits erläutert wurde, lässt sich die Trennung zwischen dem Licht zu Beleuchtungszwecken und dem Licht für die Informationsübertragung dann besonders gut erzielen, wenn zwischen den verwendeten Wellenlängen ein deutlicher Abstand besteht. Dies wiederum bedeutet jedoch, dass das Licht zu Beleuchtungszwecken bevorzugt im blauen bis violetten Bereich liegt und damit eine für die endoskopische Beobachtung ungeeignete Farbe hat. Indem das Licht auf einen Lumineszenzwandler geführt wird, der insbesondere als Fluoreszenz- oder Phosphoreszenzwandler ausgebildet ist, erhält man aus dem blauen/violetten Licht ein weißes Licht, zumindest ein weiß-ähnliches Licht. Dieses Licht ist für Beleuchtungszwecke geeignet.

Bei einer weiteren vorteilhaften Ausgestaltung der Erfindung ist am ersten proximalen Ende der endoskopischen Anordnung eine Lichtquelle angeordnet, die dafür ausgebildet ist, Licht am ersten proximalen Ende in das Datenübertragungselement einzukoppeln.

Auf diese Weise lässt sich das Datenübertragungselement zusätzlich für eine Beleuchtungsmöglichkeit und/oder Gewinnungsmöglichkeit von elektrischer Energie am ersten distalen Ende der endoskopischen Anordnung nutzen. Die Wellenlänge des Lichts, das von der Lichtquelle ausgestrahlt wird, liegt bevorzugt zwischen 380 nm und 490 nm, insbesondere zwischen 390 nm und 430 nm. Das Datenübertragungselement ist vorteilhafterweise als Multimode-Faser ausgestaltet.

Bei einer weiteren vorteilhaften Ausgestaltung der Erfindung weist das Datenübertragungselement am ersten proximalen Ende der endoskopischen Anordnung eine Trennstelle auf, die dafür ausgebildet ist, es zu ermöglichen, die endoskopische Anordnung von einer proximalseitigen elektrischen Einheit zu trennen und/oder drehbar zu koppeln.

Diese Ausgestaltung ermöglicht eine besonders einfache Austauschbarkeit der endoskopischen Anordnung. Wird die Trennstelle im Datenübertragungselement angeordnet, so kann die Trennstelle dann besonders einfach realisiert werden, wenn die Übertragungselemente innerhalb des Datenübertragungselements koaxial ausgebildet sind. Es kann so die Drehbarkeit der endoskopischen Anordnung gegenüber der proximalseitigen elektrischen Einheit erzielt werden. Dadurch ergibt sich eine besonders gute Bedienbarkeit.

Bei einer weiteren vorteilhaften Ausgestaltung der Erfindung ist das Datenübertragungselement als Monomode-Faser ausgebildet.

Diese Ausgestaltung ermöglicht einen besonders geringen Durchmesser des Datenübertragungselements. Der Faserkern einer Monomode-Faser beträgt üblicherweise weniger als 10 µm. Außerdem weisen Monomode-Fasern eine geringe Dämpfung auf. Als optimale Übertragungswellenlänge werden derzeit 1550 nm ± 200 nm, bevorzugt 1550 nm ± 100 nm und besonders bevorzugt 1550 nm ± 50 nm angesehen. Die endoskopische Anordnung ermöglicht so minimale Durchmesserdimensionen für die endoskopische HD-Bilderfassung und -übertragung, die bisher nicht realisiert werden konnten. Der Lichtwellenleiter liegt insbesondere bei einem Durchmesser von weniger als 0,1 mm.

Bei einer weiteren vorteilhaften Ausgestaltung der Erfindung ist die bildgebende Einrichtung dafür ausgebildet, die Ausgangssignale gemäß der Spezifikation MIPI D-PHY oder MIPI M-PHY zu übertragen.

Diese Ausgestaltung nutzt ein geräteinternes, serielles Standard-Übertragungsformat aus dem mobilen Kommunikationsbereich, welches sich jedoch nur für kurze Leitungslängen von weniger als 30 cm einsetzen lässt und daher in der Endoskopie bisher unbekannt ist. Da die vorgeschlagene endoskopische Anordnung elektrische Leitungen aber nur bis zum ersten Signalwandler bzw. bis zum elektrooptischen Wandler benötigt, und der größte Teil der Übertragungsstrecke über das als Lichtleiter ausgebildete Datenübertragungselement nahezu dämpfungsfrei erfolgt, kann der vorteilhafte Standard nun überraschenderweise in der Endoskopie eingesetzt werden. Aufgrund der optischen Übertragung können die Übertragungslängen in einem breiten Bereich beliebig variiert und weit über die Grenze von 30 cm hinaus erhöht werden, ohne dass sich das hochfrequente Signal zum Beispiel durch Dämpfung signifikant verändert. Die Verwendung dieses Standards ermöglicht es außerdem, eine Vielzahl von kostengünstigen Bildsensoren nutzen zu können. Die Verwendung von kostspieligen proprietären Bildsensoren entfällt dadurch. Bei der Spezifikation MIPI M-PHY handelt es sich um einen ähnlichen kommenden Standard, der im mobilen Kommunikationsbereich Einzug halten wird.

Bei einer weiteren vorteilhaften Ausgestaltung der Erfindung ist der erste Signalwandler dafür ausgebildet, das digitale kombinierte Ausgangssignal in mindestens zwei Fasern des Datenübertragungselements einzukoppeln.

Auf diese Weise kann eine besonders hohe Zuverlässigkeit der endoskopischen Anordnung gewährleistet werden. Fällt eine der Fasern des Datenübertragungselements aufgrund eines Defekts aus, so wird die vollständige Bildinformation weiterhin über die verbleibende Faser bzw. die verbleibenden Fasern übertragen. Wenn der proximalseitige optoelektrische Wandler und/oder der zweite Signalwandler dafür ausgebildet sind, die Funktionstüchtigkeit der Fasern des Datenübertragungselements zu überprüfen und den Ausfall einer Faser zu erkennen, kann der Mediziner auf die Notwendigkeit einer Reparatur oder eines Austauschs hingewiesen werden, ohne dass eine aktuelle Intervention unterbrochen werden muss.

Bei einer bevorzugten Ausgestaltung der Erfindung sind der optoelektrische Wandler und/oder der zweite Signalwandler über einen elektrischen Verbinder an die elektrische Einheit koppelbar.

Bei dieser Ausgestaltung handelt es sich um eine kostengünstige Möglichkeit, um ein einfaches Auswechseln der endoskopischen Anordnung zu ermöglichen. Endoskopische Anordnungen - wie dargestellt - können in erster Linie als flexible oder starre Videoendoskope oder Videokatheter realisiert sein, die im distalen Bereich die bildgebende Einheit tragen. Es kann sich aber auch um endoskopische Kameras handeln, die an konventionelle Endoskope gekoppelt werden und die in ihrem distalen Teil leicht und klein in ihrer Bauform auszuführen sind.

Bei einer weiteren vorteilhaften Ausführungsform ist die endoskopische Anordnung Teil einer endoskopischen Kapsel, wobei die Kapsel einen Kapselkörper, in dem die distalseitigen Elemente der endoskopischen Anordnung eingeschlossen sind, und ein Einführsystem aufweist.

Eine solche endoskopische Kapsel bietet eine geringe Größe bei gleichzeitig hoher Bildqualität.

Bei einer weiteren vorteilhaften Ausgestaltung der Erfindung ist die endoskopische Anordnung Teil einer endoskopischen Kapsel, wobei das Datenübertragungselement proximalseitig als Rolle auf einer Rolleinrichtung gewickelt ist.

Auf diese Weise kann die endoskopische Kapsel besonders einfach appliziert werden, ohne die Gefahr, dass sich das Datenübertragungselement verheddert. Da das Datenübertragungselement - wie beschrieben - einen Durchmesser von weniger als einem Millimeter aufweist und neben der Signalübertragung auch gleichzeitig zur Licht- und Energieversorgung verwendet werden kann, stellt es ein sehr geeignetes, nach heutiger Einschätzung optimales, Verbindungselement dar, ohne den Bewegungsablauf der Kapsel im Gastrointestinaltrakt zu behindern.

Bei einer weiteren vorteilhaften Ausgestaltung der Erfindung ist die endoskopische Anordnung Teil einer endoskopischen Videokamera zur Untersuchung von Hohlorganen oder Hohlräumen. Das Datenübertragungselement ist dabei als Hybridkabel ausgeführt, das sowohl den Lichtwellenleiter für die Datenübertragung als auch elektrische Zuleitungen zur Spannungsversorgung und für niederfrequente Steuersignale trägt.

Bei einer weiteren vorteilhaften Ausgestaltung der Erfindung ist die endoskopische Anordnung Teil eines Videoendoskops, wobei im Datenübertragungselement ein Steckersystem integriert ist, wodurch es möglich ist, zum Beispiel die optische Signalleitung am Endoskopgriff oder distalseitig in der Nähe des Bildsensors oder proximalseitig an einem Lichtleitstecker zum Beispiel zu Reinigungszwecken zu trennen.

Bei einer weiteren vorteilhaften Ausgestaltung der Erfindung ist die endoskopische Anordnung lösbar an einem Endoskopkörper angeordnet.

Bei einer weiteren vorteilhaften Ausgestaltung der Erfindung ist die endoskopische Anordnung Teil eines Laryngoskops, insbesondere eines Einweg-Laryngoskops, wobei das Datenübertragungselement bis in einen Handgriff des Laryngoskops führt und dort zusätzlich zu dem optoelektrischen Wandler und dem zweiten Signalwandler ein Datenkompressor und ein drahtloser Übertrager (Wireless Transmitter, zum Beispiel WIFI, WHDI, WirelessHD, UWB, WiGig, etc.), der eine drahtlose Kommunikation der Bilddaten zur Versorgungseinheit bzw. zu einem Monitor ermöglicht, integriert sind.

Bei einer weiteren vorteilhaften Ausgestaltung der Erfindung ist die endoskopische Anordnung in ein starres oder flexibles Endoskop integriert, wobei das Datenübertragungselement bis in den proximalseitigen Endoskopgriff reicht, wobei in den Endoskopgriff ein optischer Wandler, ein Datenkompressor und ein drahtloser Übertrager (Wireless Transmitter, WIFI) angeordnet sind.

Bei einer weiteren vorteilhaften Ausgestaltung der Erfindung ist die endoskopische Anordnung Teil einer endoskopischen Kapsel zur Untersuchung des Gastrointestinal-Trakts oder anderer Hohlorgane oder Hohlräume, wobei die Kapsel eine Batterie zur distalen Energieversorgung der elektrischen Komponenten aufweist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein erstes Ausführungsbeispiel einer endoskopischen Anordnung;
- Fig. 2: ein zweites Ausführungsbeispiel einer endoskopischen Anordnung;
- Fig. 3a: ein erstes Ausführungsbeispiel einer bildgebenden Einrichtung;
- Fig. 3b: ein zweites Ausführungsbeispiel einer bildgebenden Einrichtung;
- Fig. 3c: ein drittes Ausführungsbeispiel einer bildgebenden Einrichtung;
- Fig. 4: ein drittes Ausführungsbeispiel einer endoskopischen Anordnung;
- Fig. 5: ein viertes Ausführungsbeispiel einer endoskopischen Anordnung;
- Fig. 6: ein fünftes Ausführungsbeispiel einer endoskopischen Anordnung;
- Fig. 7a: ein erstes Ausführungsbeispiel einer Trennstelle für eine endoskopische Anordnung;
- Fig. 7b: ein zweites Ausführungsbeispiel einer Trennstelle für eine endoskopische Anordnung;
- Fig. 7c: ein drittes Ausführungsbeispiel einer Trennstelle für eine endoskopische Anordnung; und
- Fig. 8: ein sechstes Ausführungsbeispiel einer endoskopischen Anordnung in einer endoskopischen Kapsel.

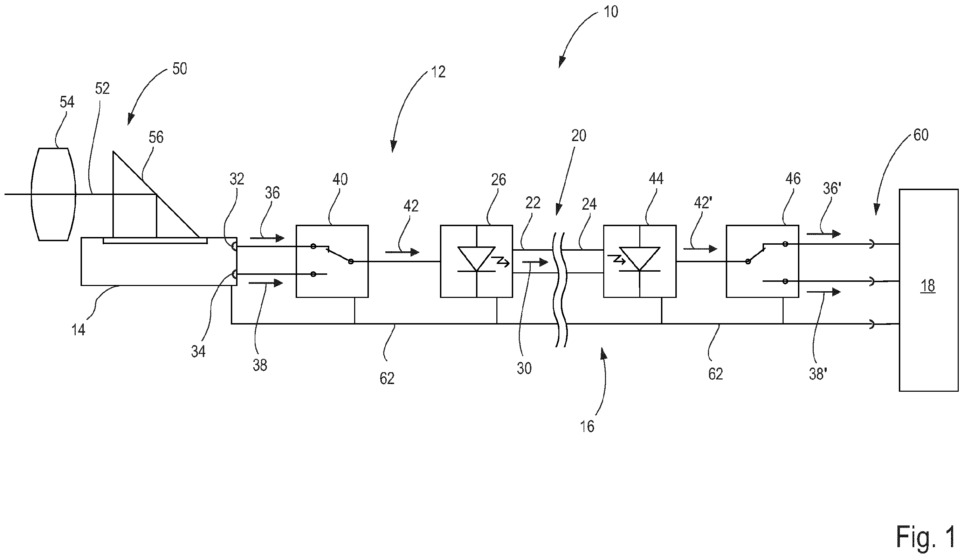

Fig. 1 zeigt eine endoskopische Anordnung 10 mit einem ersten distalen Ende 12, an dem eine bildgebende Einrichtung 14 angeordnet ist, und einem ersten proximalen Ende 16 zum Anschluss an eine elektrische Einheit 18. Die Anordnung 10 weist außerdem ein Datenübertragungselement 20 auf, das als Lichtleiter ausgebildet ist. Das Datenübertragungselement 20 hat ein zweites distales Ende 22 und ein zweites proximales Ende 24.

Das Datenübertragungselement 20 weist bevorzugt eine Länge von mindestens 1 m, besonders bevorzugt von mindestens 3 m und insbesondere von mindestens 5 m auf. Das Datenübertragungselement 20 ist bei diesem ersten Ausführungsbeispiel als Monomode-Faser ausgebildet. Die bildgebende Einrichtung 14 ist hier dafür ausgebildet, die digitalen Ausgangssignale 36, 38 gemäß der Spezifikation MIPI D-PHY oder MIPI M-PHY zu übertragen. Die gleiche Spezifikation gilt für die Signale 36', 38'.

Die Anordnung 10 weist außerdem einen elektrooptischen Wandler 26 auf, der dafür ausgebildet ist, digitale Daten, die von der bildgebenden Einrichtung 14 erzeugt werden, in ein optisches Signal 30 zu wandeln. Das Datenübertragungselement 20 ist an seinem zweiten distalen Ende 22 optisch mit dem elektrooptischen Wandler 26 gekoppelt, um das optische Signal 30 in das Datenübertragungselement 20 einzukoppeln.

Die bildgebende Einrichtung 14 weist mindestens zwei Datenausgänge 32, 34 mit jeweils einem digitalen Ausgangssignal 36, 38 auf. Mit anderen Worten sind die Datenausgänge 32, 34 jeweils dafür ausgebildet, ein digitales Ausgangssignal 36, 38 auszugeben. Zwischen der bildgebenden Einrichtung 14 und dem elektrooptischen Wandler 26 ist ein erster Signalwandler 40 angeordnet, der hier als Multiplexer ausgeführt ist. Der erste Signalwandler 40 ist dafür ausgebildet, die digitalen Ausgangssignale 36, 38 zu einem digitalen kombinierten Ausgangssignal 42 zusammenzuführen.

Die bildgebende Einrichtung 14 hat eine Videoauflösung von mehr als 0,75 Megapixel, bevorzugt von mehr als 1,5 Megapixel, besonders bevorzugt von mehr als 2 Megapixel (Full-HD) und insbesondere von mehr als 8 Megapixel (Quad-HD) bei einer Bildrate von bevorzugt mindestens 15 Bildern pro Sekunde, besonders bevorzugt mindestens 25 Bildern pro Sekunde und insbesondere von mindestens 30 Bildern pro Sekunde.

Für sogenannte Quad-HD-Systeme existieren bereits Produktankündigungen für CMOS-Bildsensoren relativ kleiner Baugröße mit Bildauflösungen über 8 Megapixel, wie z.B. dem Bildsensor OV10810 der Firma Omnivision, St. Barbara, USA. Diese für die Endoskopie einsetzbaren Bildsensoren erfordern gegenüber Full-HD eine weitere Vervierfachung der Übertragungsrate. Bei Standard-Bildfrequenzen von mindestens 25 Bildern pro Sekunde ergeben sich damit sehr hohe Übertragungsraten die mehrere GBit/s betragen. Diese sind bei endoskopischen Rahmenbedingungen nach kleinen Übertragungssystemdurchmessern in vorteilhafter Weise mit der aufgezeigten endoskopischen Anordnung in optimaler Weise umsetzbar.

Am zweiten proximalen Ende 24 des Datenübertragungselements 20 ist ein erster optoelektrischer Wandler 44 angeordnet. Der optoelektrische Wandler 44 ist dafür ausgebildet, das optische Signal 30 aus dem Datenübertragungselement 20 auszukoppeln und das optische Signal 30 in digitale Daten zu wandeln. Der erste optoelektrische Wandler 44 ist mit einem zweiten Signalwandler 46 verbunden, der dafür ausgebildet ist, ein digitales kombiniertes Ausgangssignal 42' in mindestens zwei digitale Ausgangssignale 36', 38' aufzuteilen. Es sei daraufhingewiesen, dass der optoelektrische Wandler 44 und der zweite Signalwandler 46 in die elektrische Einheit 18 integriert sein können.

Die Kennzeichnung der Signale in 36 und 36', 38 und 38' bzw. 42 und 42' zeigt an, dass es sich zwar aufgrund der Zwischenschritte bei der Signalübertragung nicht physikalisch um dasselbe Signal handelt, die distalseitig gesendeten Informationen aber in den proximalseitig empfangenen Informationen enthalten sind.

Der bildgebenden Einrichtung 14 ist eine optische Anordnung 50 zugeordnet, die einen optischen Weg mit einer optischen Achse 52 für distalseitig einfallendes Licht zur bildgebenden Einrichtung 14 bereitstellt. Die optische Anordnung 50 weist hier eine bikonvexe Linse 54 und ein Prisma 56 auf. Bei diesem Ausführungsbeispiel ist die endoskopische Anordnung 10 einschließlich des optoelektrischen Wandlers 44 und des zweiten Signalwandlers 46, bei dem es sich hier um einen De-Multiplexer handelt, über einen elektrischen Verbinder 60 mit der elektrischen Einheit 18 verbunden. Wie in der Figur angedeutet ist, ist die Verbindung lösbar. Es besteht daher eine einfache Möglichkeit, die endoskopische Anordnung 10 gegen eine andere Anordnung zu tauschen.

Außerdem ist eine elektrische Versorgungsleitung 62 gezeigt, die die distalseitigen und proximalseitigen elektrischen Komponenten mit Energie versorgt.

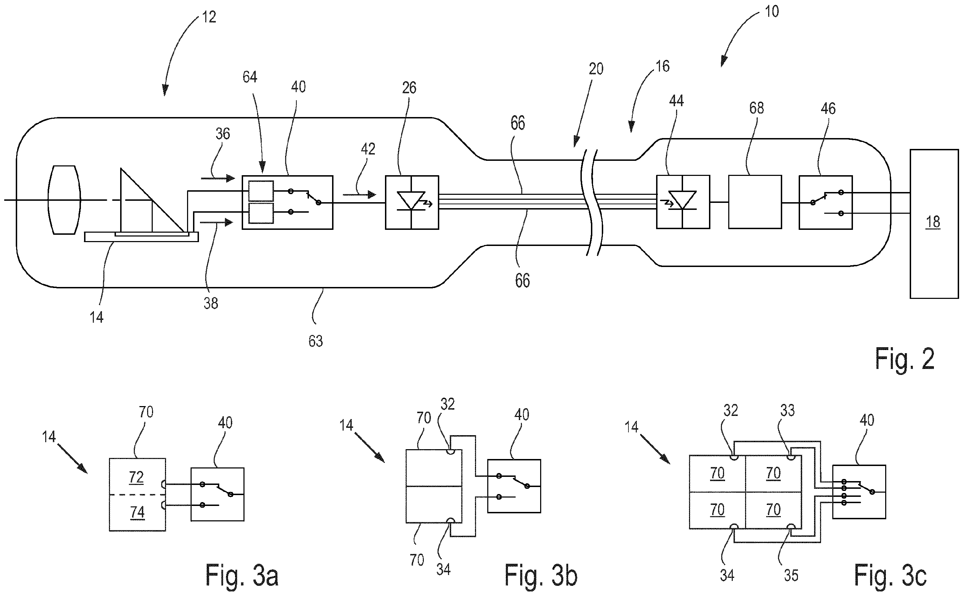

Fig. 2 zeigt ein zweites Ausführungsbeispiel einer endoskopischen Anordnung 10. Die Erläuterungen zur Funktionsweise, die bereits im Hinblick auf das erste Ausführungsbeispiel erfolgt sind, sollen hier nicht wiederholt werden und gelten auch für diese und die nachfolgenden Figuren. Der Übersichtlichkeit halber werden auch nicht alle Bezugszeichen dargestellt. Vielmehr gelten die Bezugszeichen, die bei der Erläuterung des ersten Ausführungsbeispiels eingeführt wurden, auch für diese und die nachfolgenden Figuren.

Im Unterschied zur Fig. 1 ist die endoskopische Anordnung 10 bei dem zweiten Ausführungsbeispiel von einer Umhüllung 63 umgeben. Die Umhüllung 63 schützt die endoskopische Anordnung vor äußeren Einflüssen, insbesondere vor Feuchtigkeit.

Der erste Signalwandler 40 weist bei diesem Ausführungsbeispiel einen Pufferspeicher 64 auf, der dafür ausgebildet ist, zumindest eines der digitalen Ausgangssignale 36, 38 - hier beide digitalen Ausgangssignale 36, 38 - temporär zu speichern. Dadurch lässt sich das digitale kombinierte Ausgangssignal 42 besonders flexibel zusammenführen.

Das Datenübertragungselement 20 weist bei dem zweiten Ausführungsbeispiel mindestens zwei Fasern 66 auf. Der erste Signalwandler 40 ist dafür ausgebildet, das digitale kombinierte Ausgangssignal 42 in die zwei Fasern 66 des Datenübertragungselements 20 einzukoppeln. Die Einkopplung in die zwei Fasern 66 kann bevorzugt erfolgen, indem über eine Lichtquelle in dem elektrooptischen Wandler 26, die insbesondere als Laserdiode ausgeführt ist, Licht in beide Fasern 66 eingekoppelt wird. Besonders bevorzugt im Sinne der Sicherheit kann es sein, die Einkopplung in die zwei Fasern 66 jeweils über eine eigene Lichtquelle zu realisieren. Auf diese Weise kann eine besonders hohe Sicherheit bei der Datenübertragung gewährleistet werden.

In diesem Zusammenhang ist es vorteilhaft, wenn die endoskopische Anordnung 10 über eine Überwachungseinheit 68 verfügt, die die Funktionstüchtigkeit der optischen Übertragungsstrecke überprüft. Fällt beispielsweise die Übertragung über eine der Fasern 66 aus oder, wenn die Signale über die einzelnen Fasern 66 getrennt ausgewertet werden, unterscheiden sich die empfangenen Signale voneinander, so kann ein Hinweis gegeben werden, dass die endoskopische Anordnung 10 überprüft und gegebenenfalls repariert oder getauscht werden sollte.

Fig. 3a zeigt ein erstes Ausführungsbeispiel einer bildgebenden Einrichtung 14. Dabei ist ein Bildsensor 70 mit einer durchgehenden Bildfläche gezeigt, wobei jedoch ein erster Teil 72 und ein zweiter Teil 74 der Bildfläche getrennt über die Datenausgänge 32 und 34 ausgelesen werden.

Fig. 3b zeigt ein zweites Ausführungsbeispiel einer bildgebenden Einrichtung 14. Hier sind zwei Bildsensoren 70 vorhanden, die jeweils über einen Datenausgang 32 bzw. 34 ausgelesen werden.

Fig. 3c zeigt ein drittes Ausführungsbeispiel einer bildgebenden Einrichtung 14. Hier sind vier Bildsensoren 70 angeordnet, die jeweils über einen Datenausgang 32, 33, 34 bzw. 35 ausgelesen werden.

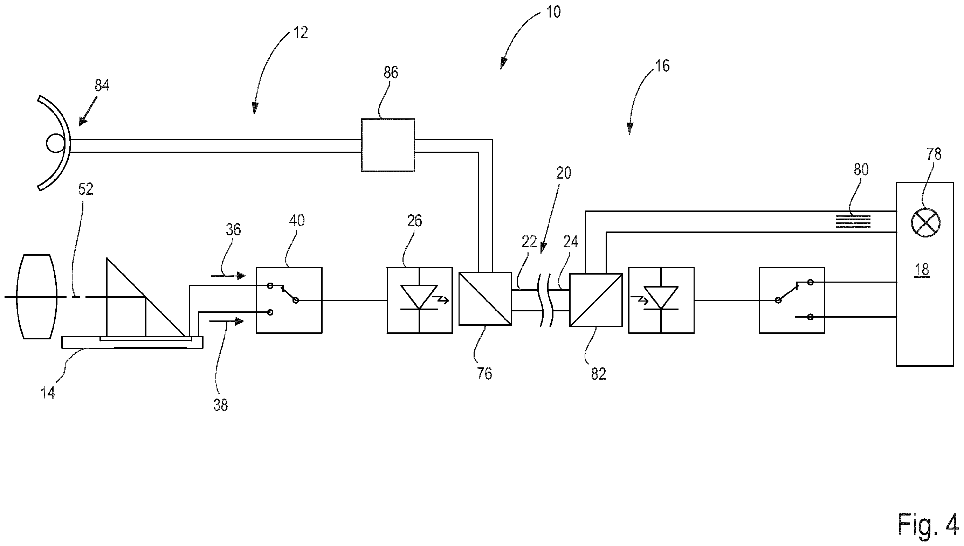

Fig. 4 zeigt ein drittes Ausführungsbeispiel einer endoskopischen Anordnung 10. Hier ist zwischen dem elektrooptischen Wandler 26 und dem zweiten distalen Ende 22 des Datenübertragungselements 20 ein Spektralteiler 76 angeordnet. Außerdem ist am ersten proximalen Ende 24 der endoskopischen Anordnung 10 eine Lichtquelle 78 angeordnet, hier in die elektrische Einheit 18 integriert, die dafür ausgebildet ist, Licht 80 am ersten proximalen Ende 24 in das Datenübertragungselement 20 einzukoppeln. Die Einkopplung findet hier über einen weiteren Spektralteiler 82 statt.

Am ersten distalen Ende 12 der endoskopischen Anordnung 10 ist ein Austritt 84 für sichtbares Licht und ein Lumineszenzwandler 86 angeordnet, wobei der Lumineszenzwandler 86 dafür ausgebildet ist, im Datenübertragungselement 20 geführtes Licht in weißes Licht zu wandeln und an den Austritt 84 weiterzuleiten.

Bei diesem dritten Ausführungsbeispiel sendet die Lichtquelle 78 blaues Licht aus, und die Lichtwellenlänge für die Übertragung der Bilddaten von der distalen Seite zur proximalen Seite liegt im infraroten, insbesondere nahinfraroten, Bereich. Die verschiedenen Wellenlängen können so von den Spektralteilern 76, 82 besonders gut getrennt werden. Am Spektralteiler 76 wird das blaue Licht in Richtung des Lumineszenzwandlers 86 geleitet. Dort wird es in weißes Licht gewandelt, zumindest in weiß-ähnliches Licht, und dann zum Austritt 84 geleitet.

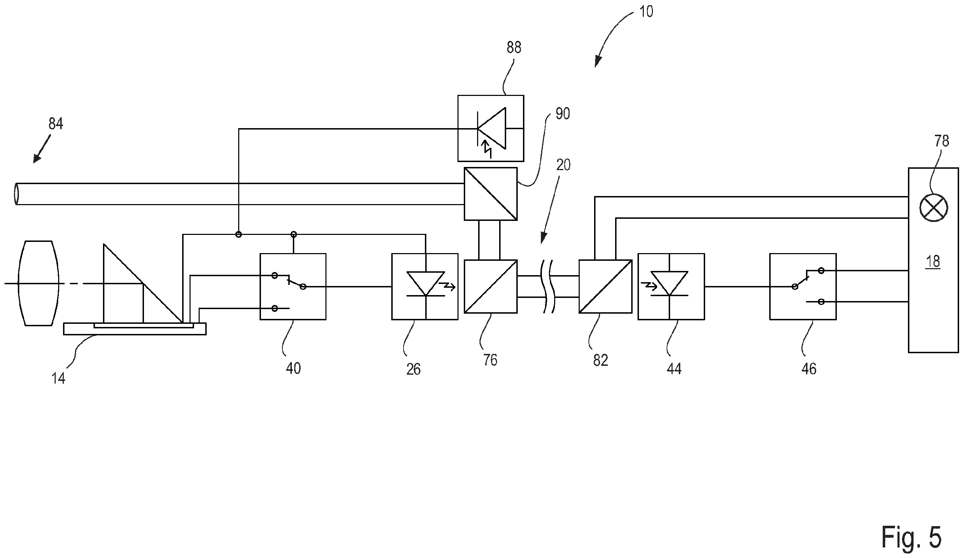

Fig. 5 zeigt ein viertes Ausführungsbeispiel einer endoskopischen Anordnung 10. Hier ist am zweiten distalen Ende 22 der endoskopischen Anordnung 10 ein zweiter optoelektrischer Wandler 88 angeordnet, der dafür ausgebildet ist, Licht, das proximalseitig in das Datenübertragungselement 20 eingekoppelt wird, in elektrische Energie zu wandeln. Die Auskopplung des Lichts, das zum zweiten optoelektrischen Wandler 88 geführt wird, wird hier mittels eines teildurchlässigen Spiegels 90 ausgekoppelt. Die elektrische Energie wird zur Versorgung der distalseitigen elektrischen Komponenten, hier die bildgebende Einrichtung 14, der erste Signalwandler 40 und der elektrooptische Wandler 26, verwendet.

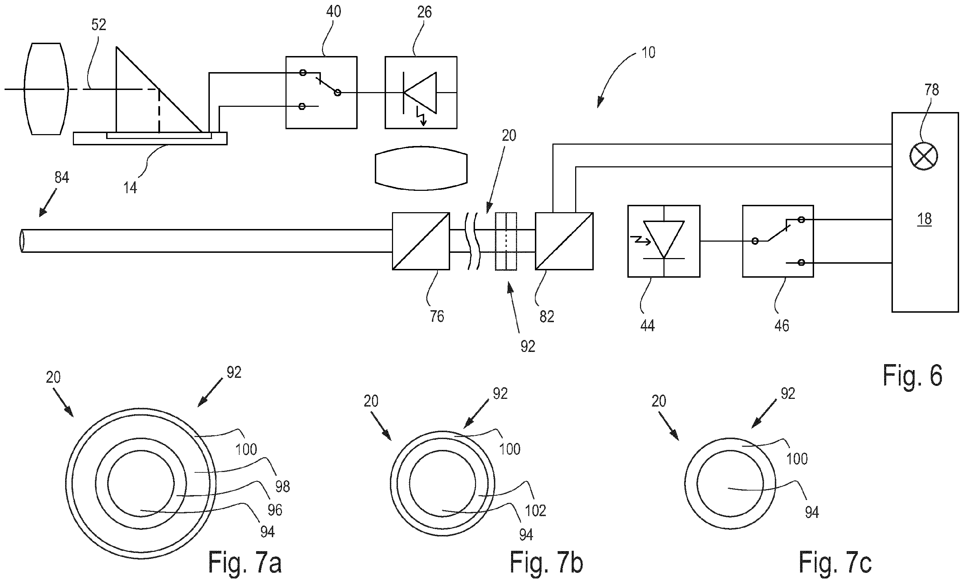

Fig. 6 zeigt ein fünftes Ausführungsbeispiel einer endoskopischen Anordnung 10. Das Datenübertragungselement 20 weist am ersten proximalen Ende 16 der endoskopischen Anordnung 10 eine Trennstelle 92 auf, die dafür ausgebildet ist, es zu ermöglichen, die endoskopische Anordnung 10 von einer proximalseitigen elektrischen Einheit 18 zu trennen.

Fig. 7a zeigt ein erstes Ausführungsbeispiel einer Trennstelle 92 im Querschnitt. Im Kern des Datenübertragungselements 20 wird eine Faser 94 für die Informationsübertragung von der distalen Seite zur proximalen Seite angeordnet. Die Faser 94 ist von einer Reflexionsschicht 96 umgeben. Die Reflexionsschicht 96 ist von einer weiteren Faser bzw. Fasern 98 umgeben, in der sichtbares Licht von der proximalen Seite zur distalen Seite geführt wird. Wie bereits erläutert, ist es aber auch möglich, das sichtbare Licht durch die Faser 94 zu führen, wenn die Faser 94 als Multimode-Faser ausgeführt ist. Schließlich hat das Datenübertragungselement 20 eine Umhüllung 100. Der gezeigte Aufbau erlaubt eine vollständige Drehbarkeit der endoskopischen Anordnung gegenüber der elektrischen Einheit 18.

Fig. 7b zeigt ein zweites Ausführungsbeispiel einer Trennstelle 92, bei dem das Licht zur Informationsübertragung und das sichtbare Licht in einer Faser 94 mit Reflexionsschicht übertragen werden. Die Faser 94 ist von einem elektrischen Leiter 102 umgeben, den wiederum eine Umhüllung 100 umgibt. Die elektrische Verbindung an der Trennstelle 92 kann beispielsweise über einen Schleifring realisiert werden. So bleibt auch bei dieser Ausgestaltung eine vollständige Drehbarkeit der endoskopischen Anordnung 10 gegenüber der elektrischen Einheit 18 gewährleistet.

Fig. 7c zeigt ein drittes Ausführungsbeispiel einer Trennstelle 92. Hier ist die Faser 94 mit Reflexionsschicht von der Umhüllung 100 umgeben. Eine solche Trennstelle 92 kann insbesondere in dem Ausführungsbeispiel gemäß Fig. 5 eingesetzt werden. Dabei überträgt die Faser 94 die Bildinformationen von der distalen Seite zur proximalen Seite und sichtbares Licht von der proximalen Seite zur distalen Seite. Das Licht wird proximalseitig sowohl als Beleuchtungslicht als auch zur Gewinnung von elektrischer Energie verwendet.

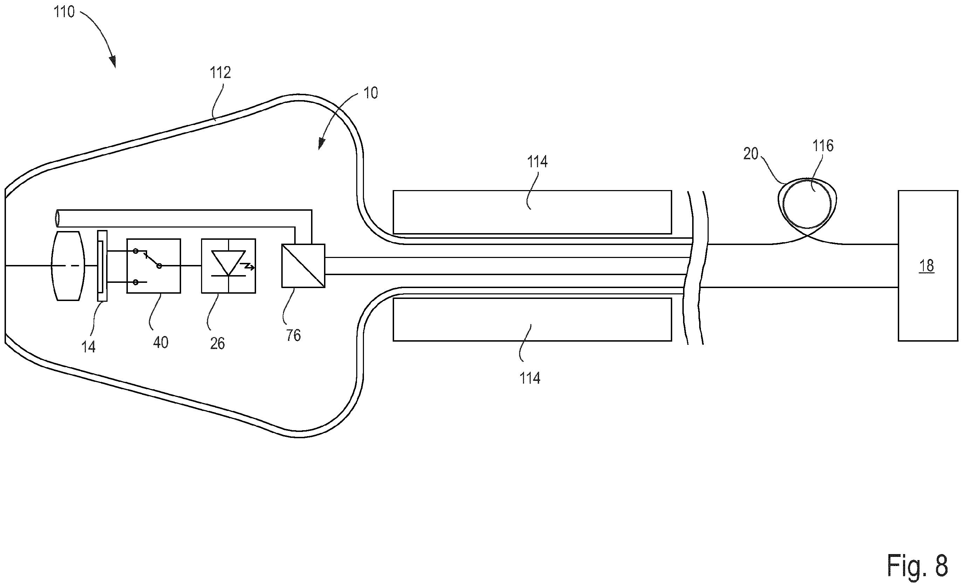

Fig. 8 zeigt eine endoskopische Kapsel 110 mit einer endoskopischen Anordnung 10. Die endoskopische Anordnung 10 ist hier in einem Kapselkörper 112 eingeschlossen, insbesondere die distalseitigen Elemente der endoskopischen Anordnung. Außerdem weist die endoskopische Kapsel 110 ein Einführsystem 114 auf. Das Datenübertragungselement 20 ist proximalseitig als Rolle auf einer Rolleinrichtung 116 gewickelt. Die Rückgewinnung aus dem optischen Signal erfolgt hier in der elektrischen Einheit 18.

Insgesamt wurde eine endoskopische Anordnung aufgezeigt, die trotz der erheblichen räumlichen Beschränkungen bei Endoskopieanwendungen die zuverlässige Übertragung von hoch- bzw. höchstauflösenden Videobildern ermöglicht.

## Patentansprüche

1. Endoskopische Anordnung (10) mit einem ersten distalen Ende (12), an dem eine bildgebende Einrichtung (14) angeordnet ist, und einem ersten proximalen Ende (16) zum Anschluss an eine Versorgungseinheit (18), mit einem Datenübertragungselement (20), das als Lichtleiter ausgebildet ist und ein zweites distales Ende (22) und ein zweites proximales Ende (24) aufweist, und mit einem elektrooptischen Wandler (26), der dafür ausgebildet ist, digitale Daten, die von der bildgebenden Einrichtung (14) erzeugt werden, in ein optisches Signal (30) zu wandeln und das Datenübertragungselement (20) an seinem zweiten distalen Ende (22) optisch mit dem elektrooptischen Wandler (26) gekoppelt ist, um das optische Signal (30) in das Datenübertragungselement (20) einzukoppeln, wobei die bildgebende Einrichtung (14) mindestens zwei Datenausgänge (32, 34) mit jeweils einem digitalen Ausgangssignal (36, 38) aufweist, und zwischen der bildgebenden Einrichtung (14) und dem elektrooptischen Wandler (26) ein erster Signalwandler (40) angeordnet ist, der dafür ausgebildet ist, die digitalen Ausgangssignale (36, 38) zu einem digitalen kombinierten Ausgangssignal (42) zusammenzuführen, wobei am zweiten proximalen Ende (24) des Datenübertragungselements (20) ein erster optoelektrischer Wandler (44) angeordnet ist, der dafür ausgebildet ist, das optische Signal (30) aus dem Datenübertragungselement (20) auszukoppeln und das optische Signal (30) in digitale Daten zu wandeln, **dadurch gekennzeichnet, dass** mit dem ersten optoelektrischen Wandler (44) ein zweiter Signalwandler (46) verbunden ist, der dafür ausgebildet ist, ein digitales kombiniertes Ausgangssignal (42') des ersten optoelektrischen Wandlers (44) in mindestens zwei digitale Ausgangssignale (36', 38') aufzuteilen, die immer noch jeweils dieselben Informationen beinhalten, wie das distalseitige digitale kombinierte Ausgangssignal.

2. Endoskopische Anordnung nach Anspruch 1, wobei der erste Signalwandler (40) einen Pufferspeicher (64) aufweist, der dafür ausgebildet ist, zumindest eines der digitalen Ausgangssignale (36, 38), bevorzugt alle digitalen Ausgangssignale, temporär zu speichern.

3. Endoskopische Anordnung nach einem der vorhergehenden Ansprüche, wobei die bildgebende Einrichtung (14) eine Videoauflösung von mehr als 0,75 Megapixel, bevorzugt von mehr als 1,5 Megapixel, besonders bevorzugt von mehr als 2 Megapixel und insbesondere von mehr als 8 Megapixel aufweist.

4. Endoskopische Anordnung nach einem der vorhergehenden Ansprüche, wobei zwischen dem elektrooptischen Wandler (26) und dem zweiten distalen Ende (22) des Datenübertragungselements (20) ein Spektralteiler (76) angeordnet ist.

5. Endoskopische Anordnung nach einem der vorhergehenden Ansprüche, wobei am ersten distalen Ende (12) der endoskopischen Anordnung (10) ein zweiter optoelektrischer Wandler (88) angeordnet ist, der dafür ausgebildet ist, Licht (80), das proximalseitig in das Datenübertragungselement (20) eingekoppelt wird, in elektrische Energie zu wandeln.

6. Endoskopische Anordnung nach einem der vorhergehenden Ansprüche, wobei am ersten distalen Ende (12) der endoskopischen Anordnung (10) ein Austritt (84) für sichtbares Licht und ein Lumineszenzwandler (86) angeordnet sind, wobei der Lumineszenzwandler (86) dafür ausgebildet ist, im Datenübertragungselement (20) geführtes Licht (80) in weißes Licht zu wandeln und an den Austritt (84) weiterzuleiten.

7. Endoskopische Anordnung nach einem der vorhergehenden Ansprüche, wobei am ersten proximalen Ende (16) der endoskopischen Anordnung (10) eine Lichtquelle (78) angeordnet ist, die dafür ausgebildet ist, Licht (80) am ersten proximalen Ende (16) in das Datenübertragungselement (20) einzukoppeln.

8. Endoskopische Anordnung nach einem der vorhergehenden Ansprüche, wobei das Datenübertragungselement (22) am ersten proximalen Ende (14) der endoskopischen Anordnung (10) eine Trennstelle (92) aufweist, die dafür ausgebildet ist, es zu ermöglichen, die der endoskopische Anordnung (10) von einer proximalseitigen elektrischen Einheit (18) zu trennen und/oder drehbar zu koppeln.

9. Endoskopische Anordnung nach einem der vorhergehenden Ansprüche, wobei das Datenübertragungselement (20) als Monomode-Faser ausgebildet ist.

10. Endoskopische Anordnung nach einem der vorhergehenden Ansprüche, wobei die bildgebende Einrichtung (14) dafür ausgebildet ist, die digitalen Ausgangssignale (36, 38) gemäß der Spezifikation MIPI D-PHY oder MIPI M-PHY zu übertragen.

11. Endoskopische Anordnung nach einem der vorhergehenden Ansprüche, wobei das Datenübertragungselement (20) mindestens zwei Fasern (66) aufweist und der elektrooptische Wandler (26) dafür ausgebildet ist, das digitale kombinierte Ausgangssignal (42) in die mindestens zwei Fasern (66) des Datenübertragungselements (20) einzukoppeln.

12. Endoskopische Anordnung nach einem der vorhergehenden Ansprüche, wobei der optoelektrische Wandler (44) und/oder der zweite Signalwandler (46) über einen elektrischen Verbinder (60) an die elektrische Einheit (18) koppelbar sind.

13. Endoskopische Kapsel (110) mit einer endoskopischen Anordnung (10) nach einem der vorhergehenden Ansprüche, wobei die Kapsel (110) einen Kapselkörper (112), in der die distalseitigen Elemente der endoskopischen Anordnung (10) eingeschlossen sind, und ein Einführsystem (114) aufweist.

14. Endoskopische Kapsel, insbesondere nach Anspruch 13, mit einer endoskopischen Anordnung (10) nach einem der Ansprüche 1 bis 12, wobei das Datenübertragungselement (20) proximalseitig als Rolle auf einer Rolleinrichtung (116) gewickelt ist.

## Claims

1. Endoscopic arrangement (10) comprising a first distal end (12) at which an imaging device (14) is arranged, and a first proximal end (16) for connecting to a supply unit (18), a data transmission element (20) embodied as a light guide and having a second distal end (22) and a second proximal end (24), and comprising an electro-optical converter (26) configured to convert digital data generated by the imaging device (14) into an optical signal (30), wherein the data transmission element (20) at its second distal end (22) is optically coupled to the electro-optical converter (26) for coupling the optical signal (30) into the data transmission element (20), wherein the imaging device (14) comprises at least two data outputs (32, 34) each having a digital output signal (36, 38), and wherein a first signal converter (40) is provided between the imaging device (14) and the electro-optical converter (26) configured for combining the digital output signals (36, 38) into a digital combined output signal (42), wherein a first opto-electrical converter (44) is arranged at the second proximal end (24) of the data transmission element (20) configured to couple the optical signal (30) out of the data transmission element (20) and to convert the optical signal (30) into digital data, **characterized in that** a second signal converter (46) is connected to the first opto-electrical converter (44) which is configured to separate a digital combined output signal (42') of the first opto-electrical converter (44) into at least two digital output signals (36', 38') which still contain the same information as the digital combined output signal at the distal side.

2. Endoscopic arrangement according to claim 1, wherein the first signal converter (40) comprises a buffer memory (64) configured to temporarily store at least one of the digital output signals (36, 38), preferably all digital output signals.

3. Endoscopic arrangement according to any preceding claim, wherein the imaging device has a resolution of more than 0.75 megapixel, preferably more than 1.5 megapixel, more preferably of more than 2 megapixel and in particular more than 8 megapixel.

4. Endoscopic arrangement according to any preceding claim, wherein a spectral divider (76) is arranged between the electro-optical converter (26) and the second distal end (22) of the data transmission element.

5. Endoscopic arrangement according to any preceding claim, wherein a second opto-electrical converter (88) is arranged at the first distal end (12) of the endoscopic arrangement (10) which is configured to convert light (80) that is coupled into the data transmission element (20) at the proximal side into electric energy.

6. Endoscopic arrangement according to any preceding claim, wherein an exit (84) for visible light and a luminescence converter (86) are arranged at the first distal end (12) of the endoscopic arrangement (10), wherein the luminescence converter (86) is configured to convert light (80) which is guided in the data transmission element (20) into white light and guide it towards the exit (84).

7. Endoscopic arrangement according to any preceding claim, wherein a light source (78) is arranged at the first proximal end (16) of the endoscopic arrangement (10) which is configured to couple light (80) at the first proximal end (16) into the data transmission element (20).

8. Endoscopic arrangement according to any preceding claim, wherein the data transmission element (22) comprises a separation section (92) at the first proximal end (14) of the endoscopic arrangement (10) which is configured to allow to separate and/or to rotatably couple the endoscopic arrangement (10) relative to an electrical unit (18) at the proximal side.

9. Endoscopic arrangement according to any preceding claim, wherein the data transmission element (20) is embodied as a monomode-fiber.

10. Endoscopic arrangement according to any preceding claim, wherein the imaging device (14) is configured to transmit the digital output signals (36, 38) according to the MIPI D-PHY or MIPI M-PHY specification.

11. Endoscopic arrangement according to any preceding claim, wherein the data transmission element (20) comprises at least two fibers (66) and wherein the electro-optical converter (26) is configured to couple the digital combined output signals (42) into the at least two fibers (66) of the data transmission element (20).

12. Endoscopic arrangement according to any preceding claim, wherein the optoelectrical converter (44) and/or the second signal converter (46) are coupleable to the electrical unit (18) via an electrical connector (60).

13. Endoscopic capsule (110) comprising an endoscopic arrangement (10) according to any preceding claim, wherein the capsule (110) comprises a capsule body (112) into which the distal elements of the endoscopic arrangement (10) are enclosed, and an insertion system (114).

14. Endoscopic capsule, in particular according to claim 13, comprising an endoscopic arrangement (10) according to any of claims 1 to 12, wherein the data transmission element (20) is wound as a roll on a roller (116) at the proximal side.

## Revendications

1. Système endoscopique (10) comportant une première extrémité distale (12), sur laquelle est disposé un dispositif d'imagerie (14), et une première extrémité proximale (16) devant être raccordée à une unité d'alimentation (18), comportant un élément de transmission de données (20) qui est réalisé sous la forme d'un conducteur de lumière et une deuxième extrémité distale (22) et comportant une deuxième extrémité proximale (24), et muni d'un convertisseur électrooptique (26) qui est réalisé pour convertir des données numériques générées par le dispositif d'imagerie (14), en un signal optique (30) et l'élément de transmission de données (20) sur sa deuxième extrémité distale (22) est couplé optiquement au convertisseur électrooptique (26), afin de coupler en entrée le signal optique (30) dans l'élément de transmission de données (20), dans lequel le dispositif d'imagerie (14) comporte au moins deux sorties de données (32, 34) ayant respectivement un signal numérique de sortie (36, 38) et, entre le dispositif d'imagerie (14) et le convertisseur électrooptique (26), est disposé un premier convertisseur de signal (40) qui est réalisé pour rassembler les signaux de sortie numériques (36, 38) en un signal numérique de sortie combiné (42), dans lequel, à la deuxième extrémité proximale (24) de l'élément de transmission de données (20), est disposé un premier convertisseur optoélectrique (44) qui est réalisé pour coupler le signal optique (30) en sortie de l'élément de transmission de données (20) et pour convertir le signal optique (30) en des données numériques, **caractérisé en ce qu'**un deuxième convertisseur de signal (46) est relié au premier convertisseur optoélectrique (44), lequel deuxième convertisseur de signal est réalisé pour diviser un signal numérique de sortie combiné (42') du premier convertisseur optoélectrique (44) en au moins deux signaux numériques de sortie (36', 38'), qui contiennent encore respectivement les mêmes informations que le signal numérique de sortie combiné côté distal.

2. Système endoscopique selon la revendication 1, dans lequel le premier convertisseur de signal (40) comporte une mémoire tampon (64) qui est conçue pour stocker temporairement au moins l'un des signaux numériques de sortie (36, 38), de préférence tous les signaux numériques de sortie.

3. Système endoscopique selon l'une quelconque des revendications précédentes, dans lequel le dispositif d'imagerie (14) présente une résolution vidéo de plus de 0,75 mégapixels, de préférence de plus de 1,5 mégapixels, de manière particulièrement préférée de plus de 2 mégapixels et notamment de plus de 8 mégapixels.

4. Système endoscopique selon l'une quelconque des revendications précédentes, dans lequel un diviseur spectral (76) est disposé entre le convertisseur électrooptique (26) et la deuxième extrémité distale (22) de l'élément de transmission de données (20).

5. Système endoscopique selon l'une quelconque des revendications précédentes, dans lequel un deuxième convertisseur optoélectrique (88) est disposé à la première extrémité distale (12) du système endoscopique (10), lequel deuxième convertisseur optoélectrique est réalisé pour convertir une lumière (80) qui est couplée en entrée côté proximal dans l'élément de transmission de données (20), en énergie électrique.

6. Système endoscopique selon l'une quelconque des revendications précédentes, dans lequel une sortie (84) destinée à de la lumière visible et un convertisseur de luminescence (86) sont disposés à la première extrémité distale (12) du système endoscopique (10), dans lequel le convertisseur de luminescence (86) est réalisé pour convertir la lumière (80) guidée dans l'élément de transmission de données (20) en lumière blanche et pour l'acheminer à la sortie (84).

7. Système endoscopique selon l'une quelconque des revendications précédentes, dans lequel, à la première extrémité proximale (16) du système endoscopique (10), est disposée une source lumineuse (78), qui est réalisée pour coupler en entrée la lumière (80) présente à la première extrémité proximale (16) dans l'élément de transmission de données (20).

8. Système endoscopique selon l'une quelconque des revendications précédentes, dans lequel l'élément de transmission de données (22) comporte à la première extrémité proximale (14) du système endoscopique (10) un point de séparation (92), qui est réalisée pour permettre de séparer le système endoscopique (10) d'une unité électrique côté proximal (18) et/ou de la coupler en rotation.

9. Système endoscopique selon l'une quelconque des revendications précédentes, dans lequel l'élément de transmission de données (20) est réalisé sous la forme d'une fibre monomode.

10. Système endoscopique selon l'une quelconque des revendications précédentes, dans lequel le dispositif d'imagerie (14) est réalisé pour transmettre les signaux de sortie numériques (36, 38) conformément à la spécification MIPI D-PHY ou MIPI MPHY.

11. Système endoscopique selon l'une quelconque des revendications précédentes, dans lequel l'élément de transmission de données (20) comporte au moins deux fibres (66) et le convertisseur électrooptique (26) est réalisé pour coupler en entrée le signal numérique de sortie combiné (42) dans les au moins deux fibres (66) de l'élément de transmission de données (20).

12. Système endoscopique selon l'une quelconque des revendications précédentes, dans lequel le convertisseur optoélectrique (44) et/ou le deuxième convertisseur de signal (46) peut être couplé à l'unité électrique (18) par l'intermédiaire d'un connecteur électrique (60).

13. Capsule endoscopique (110) comportant un système endoscopique (10) selon l'une quelconque des revendications précédentes, dans lequel la capsule (110) comporte un corps de capsule (112) dans lequel sont inclus les éléments côté distal du système endoscopique (10), et un système d'introduction (114).

14. Capsule endoscopique, notamment selon la revendication 13, comportant un système endoscopique (10) selon l'une quelconque des revendications 1 à 12, dans lequel l'élément de transmission de données (20) côté proximal est enroulé sous la forme d'un rouleau sur une dispositif à rouleau (116).
